**Europäisches Patentamt**

**European Patent Office**

**Office européen des brevets**

(19)

(11) Veröffentlichungsnummer: **0 150 035 B1**

# EUROPÄISCHE PATENTSCHRIFT

(12)

(45) Veröffentlichungstag der Patentschrift: **17.04.91**

(51) Int. Cl.⁵: **G01P 3/36, G02B 27/48**

(21) Anmeldenummer: **85100355.8**

(22) Anmeldetag: **15.01.85**

(54) Verfahren, Schaltungsanordnung und Einrichtung zur berührungslosen real-time-Bestimmung von Geschwindigkeiten sowie deren Verwendung.

(30) Priorität: **18.01.84 DE 3401535**

(43) Veröffentlichungstag der Anmeldung:
**31.07.85 Patentblatt 85/31**

(45) Bekanntmachung des Hinweises auf die
Patenterteilung:
**17.04.91 Patentblatt 91/16**

(84) Benannte Vertragsstaaten:
**AT BE CH DE FR GB IT LI NL SE**

(56) Entgegenhaltungen:
**DE-A- 2 730 466**
**DE-A- 3 242 771**

**APPLIED PHYSICS, Band 25, 1981, Seiten
187-194, Springer-Verlag; T. ASAKURA et al.:
"Dynamic laser speckles and their application to velocity measurements of the diffuse
object"**

(73) Patentinhaber: **GESELLSCHAFT FÜR
STRAHLEN- UND UMWELTFORSCHUNG
M.B.H.
Ingolstädter Landstrasse 1 Post Oberschleissheim
W-8042 Neuherberg(DE)**

(72) Erfinder: **Haina, Diether. Dr.
Im Schäfergarten 4
W-8146 Alsbach 2(DE)**
Erfinder: **Waidelich, Wilhelm. Prof. Dr.
Becker-Gundulastrasse 32
W-8000 München 70(DE)**
Erfinder: **Ruth, Bernhard. Dr.
Kirchenweg 10
W-8057 Eching-Dietersheim(DE)**

(74) Vertreter: **Gottlob, Peter
Kernforschungszentrum Karlsruhe GmbH
Stabs. Patente und Lizenzen Weberstrasse 5
W-7500 Karlsruhe 1(DE)**

## Beschreibung

Die Erfindung betrifft ein Verfahren, eine Schaltungsanordnung sowie eine Einrichtung zur berührungslosen real-time-Bestimmung von Geschwindigkeiten einer oder mehrerer Bewegungen an einem Objekt in senkrechter Richtung zu einer auf das Objekt auftreffenden kohärenten Strahlung, deren Streulicht ein räumliches Speckle-Muster mit einem Speckle-Spektrum erzeugt, wobei das Speckle-Muster durch die Bewegungen am Objekt zeitabhängig wird, sowie deren Verwendung.

Ein Laserstrahl trifft in x-Richtung auf ein Objekt und wird gestreut. Das Streulicht bildet eine granulare Struktur, die Speckles, falls der mittlere Abstand *) $\Delta x$ viel gröber als $\lambda$ ist. Das ist z.B. bei allen normalen Oberflächen der Fall. Die Intensitätsverteilung der Speckles ist regellos. Für die statistische Verteilung der Speckles bestehen bestimmte Gesetzmäßigkeiten. Diese Gesetzmäßigkeiten sind unabhängig von den Objekteigenschaften, wenn nur die Voraussetzung $\Delta x$ größer $\lambda$ erfüllt ist.

Bei ruhendem Laser und ruhendem Objekt steht das Speckle-Muster still. Bei bestimmten Voraussetzungen der gegenseitigen Position von Laser, Objekt und Beobachtungspunkt bewegt sich das Speckle-Muster als Ganzes, wenn das Objekt selbst bewegt wird. Hieraus ergibt sich eine bestimmte Speckle-Geschwindigkeit $V_s$, die in geeigneten Fällen proportional zur Objektgeschwindigkeit ist. Eine Möglichkeit, die Speckle-Bewegung zu registrieren besteht darin, die Lichtintensität des Streulichtes in einer effektiven Detektorfläche zu messen, die klein ist im Vergleich zur mittleren Speckle-Größe $\sigma$.

Es gibt nun verschiedene Möglichkeiten, aus dem zeitabhängigen Signal $I(t)$ die Speckle-Geschwindigkeit zu ermitteln. Eines der Verfahren ist das sogenannte Korrelationsverfahren. Hierbei werden an zwei räumlich getrennten Stellen die Intensitäten aufgenommen. Es entstehen zwei zeitliche Intensitätsabhängigkeiten. Beide Intensitätsabhängigkeiten werden gespeichert. Die daraus errechnete Korrelationsfunktion zeigt einen Peak, aus dessen Lage die Speckle-Geschwindigkeit ermittelt werden kann.

Der Nachteil dieser Methode besteht darin, daß ein hoher elektronischer Aufwand erforderlich ist und daß die Geschwindigkeit nicht in real-time ermittelt wird (Posey, J.Phys.D 9 (1976) 1399) . Bei diesem Verfahren wird im Prinzip keine spezielle Speckle-Eigenschaft ausgenutzt.

Für das Verfahren mit der zeitlichen Integration des Signals $I(t)$ wird nur ein einziges Signal $I(t)$ benötigt. Zunächst werden aus der Speckle-Intensität $I(t)$ durch eine Integration über eine Folge von Zeitintervallen die Mittelwerte $N_i$ gebildet, von denen wiederum mit Hilfe eines Rechners eine Standardabweichung S und ein Gesamtmittelwert $\overline{N}$ errechnet werden. Der Quotient aus beiden Werten $S/\overline{N}$ ist ein nicht-lineares Maß für die Speckle-Geschwindigkeit. Der Nachteil dieses Verfahrens besteht wiederum im hohen elektronischen Rechneraufwand und darin, daß es kein real-time-Verfahren ist. Im Prinzip wird ausgenutzt, daß der Kontrast des Speckle-Signals mit zunehmender Integrationszeit in bekannter Weise gegen O geht (Ontsubo,J. Asakura, T., Opt.Quant.Electr. 8 (1976) 523).

In einer früheren Patentanmeldung DE-A-32 42 771.9 ist das sogenannte Speckle-Counting beschrieben. Bei diesem Verfahren werden Schnittpunkte zwischen der Intensität $I(t)$ und einer Schwelle S gezählt. Die Schwelle S ist proportional zur mittleren Intensität $\overline{I}$ eingestellt. Der Vorteil des Verfahrens besteht darin, daß der elektronische Aufwand gering wird und daß das Verfahren real-time-Eigenschaften hat.

Der Nachteil des Verfahrens ist jedoch dadurch gegeben, daß dem Speckle-Signal Rauschen überlagert ist. Insbesondere bei niedrigen Geschwindigkeiten führt das Rauschen zu Fehlmessungen, weil eine zu hohe Geschwindigkeit angezeigt wird. Prinzipiell wird bei dem Verfahren die Speckle-Eigenschaft ausgenutzt, daß der zeitliche Abstand $\Delta t$ zwischen zwei Counts einen Mittelwert $\overline{\Delta t}$ hat, der durch $\Delta t$ ungefähr const $\cdot \sigma / V$ gegeben ist. Keines der bisherigen Verfahren eignet sich dazu, überlagerte Geschwindigkeiten zu trennen.

Die der Erfindung zugrundeliegende Aufgabe besteht nunmehr darin, die Bewegung des Speckle-Musters derart registrieren zu können, daß aus den erhaltenen Daten die Speckle-Geschwindigkeit zu bestimmen ist unabhängig von überlagerten anderen Geschwindigkeiten. Insbesondere soll es gelingen, Durchblutungsgeschwindigkeiten der Haut oder anderer Körperteile bestimmen zu können. Die Lösung dieser Aufgabe ist bei dem Verfahren der eingangs genannten Art mittels des kennzeichnenden Merkmals des Anspruches 1 wiedergegeben.

Die übrigen Ansprüche geben vorteilhafte Weiterbildungen des erfindungsgemäßen Verfahrens einer Schaltungsanordnung, einer Einrichtung sowie eine besondere Verwendungsmöglichkeit an.

Das erfindungsgemäße Verfahren nutzt aus, daß bei einem stehenden Objekt $I(t)$ = konstant ist. Es treten keine Frequenzen ungleich O auf. Wird das Objekt bewegt, dann treten in $I(t)$ Frequenzen ungleich O auf. Das Messen der Intensität des Signals (es handelt sich hierbei um die Intensität eines elektrischen Signals) bei Frequenzen ungleich O ist also ein Maß für die Geschwindigkeit $V_s$. Ausgenutzt wird

*)der Streuzentren

2

insbesondere, daß das Speckle-Muster überhaupt eine granuläre Struktur aufweist. Wenn dazu übergegangen wird, nicht einfach die Intensität oberhalb einer Frequenz $\gamma_0$ zu bestimmen, sondern erst das Signal geeignet zu verändern, dann entsteht durch die Intensitätsbildung ein Meßwert, der proportional zur Geschwindigkeit $V_S$ ist. Diese Maßnahme besteht darin, daß die Amplituden $A(\gamma)$ des Spektrums erst um einen Faktor proportional zu $\gamma$ zu verstärken sind.

Aufgrund der speziellen Form des Speckle-Spektrums ist die Intensität des so gebildeten Signals proportional zur Geschwindigkeit $V_S$. Die Proportionalität ist durch eine theoretische Ableitung und experimentelle Daten erwiesen.

Der Meßwert M ist wegen der Intensitätsbildung auch proportional zur Gesamtlichtintensität $\bar{I}$ am Beobachtungspunkt. Um Störungen durch Laser-Intensitätsschwankungen und Änderungen der Reflexionsbedingungen zu vermeiden, kann durch eine Division des Meßwertes durch die Gesamtlichtintensität $\bar{I}$ die Störung beseitigt werden.

Der Vorteil des Verfahrens besteht darin, daß der elektronische Aufwand zur Realisierung gering gehalten werden kann. Die frequenzabhängige Wichtung erfolgt durch eine zeitliche Differentiation und die Intensitätsbildung durch ein Power-Meter.

Es ist auch nicht unbedingt erforderlich, gleichzeitig die Gesamtintensität zu messen. Die Abhängigkeit ist im schlimmsten Fall linear mit Intensitätsvariationen. Wenn also große Geschwindigkeitsdifferenzen zu messen sind und andererseits ein stabiler Laser zur Verfügung steht, dann kann darauf verzichtet werden.

Bei dem beschriebenen Speckle-Counting (DE-A-32 42 771.9) ist das nicht der Fall. Da hängt die Zählrate sehr empfindlich von der Schwelle S und damit von der gemessenen mittleren Intensität $\bar{I}$ ab.

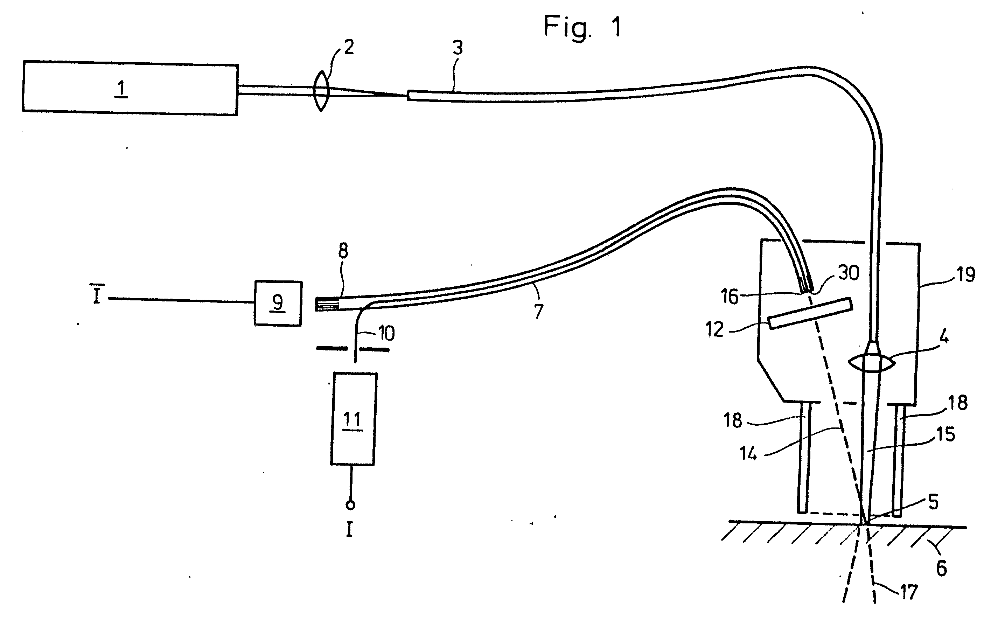

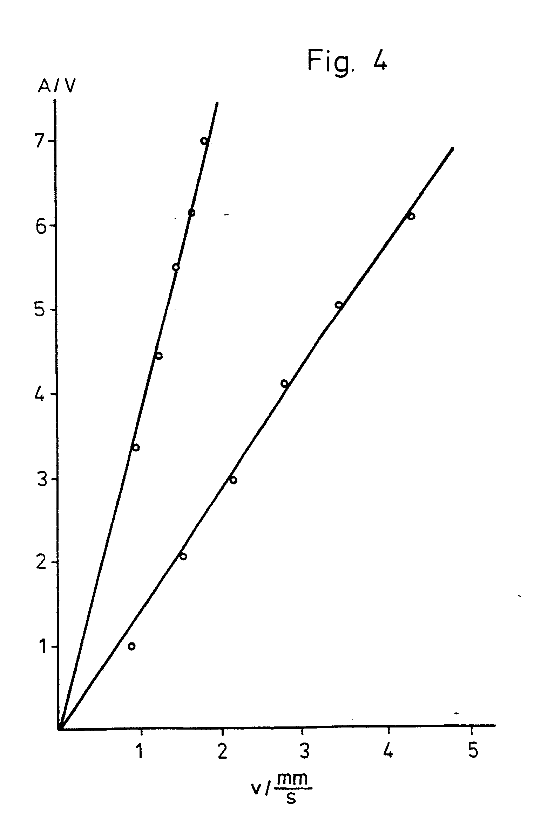

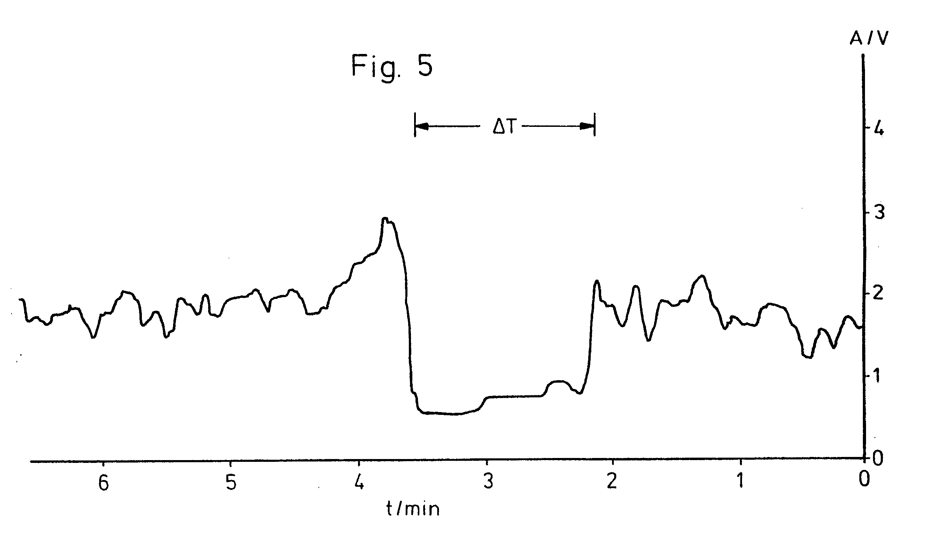

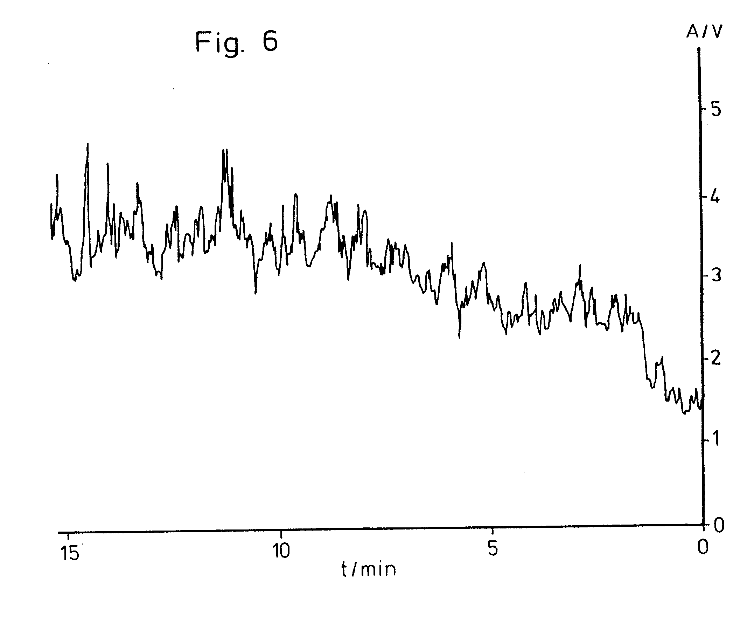

Die Erfindung wird im folgenden anhand eines Ausführungsbeispiels mittels der Figuren 1 bis 6 näher erläutert.

Bei der Speckle-Bildung kommt es darauf an, daß die Streuung des Laserlichts an Streuzentren erfolgt, die einen mittleren Abstand $\Delta x$ größer $\lambda$ aufweisen. Bei normalen bewegten Objekten besitzen alle Streuzentren die gleiche Geschwindigkeit. Bei der Haut ist dies jedoch anders; die Streuzentren können verschiedene Geschwindigkeiten haben. Das entstehende Speckle-Muster bewegt sich nun beeinflußt durch die Geschwindigkeit der Hautoberfläche und der der roten Blutzellen. Da es sich um eine kohärente Überlagerung von zwei Speckle-Mustern entsprechend den beiden Geschwindigkeiten handelt, entsteht ein neues Speckle-Muster, das sich im Aussehen nicht von dem Speckle-Muster eines normalen Objektes unterscheidet. Lediglich das zeitliche Verhalten ist ein anderes.

Mit den bisherigen bekannten Speckle-Methode ist es nicht möglich, diese beiden Geschwindigkeiten oder noch andere voneinander zu trennen. Es würde entweder einfach eine geringfügig höhere Geschwindigkeit angezeigt werden, ohne daß man unterscheiden könnte, ob das auf eine erhöhte Hautgeschwindigkeit oder eine erhöhte Blutbewegung zurückzuführen ist. Experimentell konnte jedenfalls kein Unterschied zwischen durchbluteter und nicht durchbluteter Haut festgestellt werden.

Bei den erstgenannten bekannten Verfahren besteht wahrscheinlich gar keine Möglichkeit, vom Prinzip her einen Unterschied zwischen durchbluteter und nichtdurchbluteter Haut zu finden. Selbst wenn es möglich wäre, würde es in der Praxis nicht einsetzbar sein, weil die Meßdauern sehr lang sind und der apparative Aufwand zu groß wäre.

Da der größte Teil des Lichtes an der Hautoberfläche und den Hautzellen gestreut wird, wird auch die Bewegung des Speckle-Musters hauptsächlich durch die Hautgeschwindigkeit $V_H$ bestimmt. Nur ein geringer Teil des Lichtes dringt bis in die Blutader vor, wird dort zurückgestreut und gelangt dann wieder nach außen. Der Anteil der Blutbewegung an dem entsprechenden Speckle-Muster ist daher immer gering.

Wenn ein Textilband auf die Haut geklebt und dann eine Messung durchgeführt oder aber die Blutzufuhr bei einer Hautpartie unterdrückt würde,erhielte man ungefähr einen Meßwert für die Hautgeschwindigkeit. Das Spektrum des Signals I(t) für eine solche Messung setzt sich aus einer Überlagerung von mehreren Geschwindigkeiten zusammen. Bei einer Messung an der Haut entsteht ein Spektrum, bei dem die hohen Frequenzen stärker vertreten sind im Vergleich zur Messung an nicht durchbluteter Haut. Daraus ist der Schluß zu ziehen, daß die mittlere Geschwindigkeit der Blutbewegung höher ist als die der Hautbewegung.

Grob kann das Spektrum aus zwei Teilen, dem der Hautbewegung und dem der Blutbewegung, zusammengesetzt werden. Aufgrund des Unterschieds der mittleren Geschwindigkeiten $\bar{V}_H$ und $\bar{V}_B$ ist nun eine Trennung der beiden Geschwindigkeiten möglich. Es wird nur die Intensität des Anteils des Signals gebildet, deren Frequenz oberhalb von $\gamma_g$ liegt, und der Anteil der Frequenz kleiner als $\gamma_g$ unterdrückt. Das entspräche im Spektrum einer Integration über ein Frequenzintervall $\gamma_g$ gegen unendlich.

Im Prinzip funktioniert die Trennung der beiden Geschwindigkeitsbereiche auch bei der Intensitätsbildung des (elektrischen) Signals, ohne daß eine gewichtete Verstärkung eingesetzt wird. Die frequenzabhängige Verstärkung macht den Unterschied zwischen durchbluteter und nicht durchbluteter Haut größer.

3

Eine einheitliche Definition des Blutflusses gibt es nicht. Es wird aber als sinnvoll angesehen, den Blutfluß proportional der Geschwindigkeit bzw. proportional der Blutmenge anzusetzen. Das Verfahren gestattet also eine Bestimmung des Blutflusses , denn das Signal ist proportional zur Geschwindigkeit (wegen der frequenzabhängigen Wichtung) und auch proportional zur Menge des bewegten Blutes. Tritt mehr bewegtes Blut in den Bereich der Eindringtiefe des Laserlichts ein, so wird natürlich das Signal vergrößert.

Die Figur 1 zeigt schematisch eine Meßanordnung bzw. Einrichtung, mit der die Geschwindigkeit bestimmt werden kann. Auf den Meßpunkt 5 des Objektes 6 wird ein Laserstrahl 15 gerichtet. Das Streulicht bildet aufgrund der Rauhheit des Objektes 6 eine granuläre Struktur, die Speckles, deren mittlere Größe $\sigma$ am Beobachtungsort 16 nur durch die benutzte Wellenlänge $\lambda$ des Lichts (z.B. He-Ne-Laser, 633 nm), der Strahlfleckgröße und dem Abstand vom Meßpunkt 5 bestimmt ist. Bei geeigneter optischer Anordnung bewegt sich das Speckle-Muster als Ganzes, so daß die Speckle-Geschwindigkeit $V_s$ definiert werden kann.

Für kleine Winkel zwischen dem Laserstrahl 15 und der Beobachtungsrichtung 14 gilt eine einfache Beziehung,wie sie später noch definiert wird.

Die Detektion der Speckles erfolgt mit Hilfe einer Blende 30,die zur weitgehenden Erhaltung des Speckle-Kontrastes kleiner ist als die mittlere Speckle-Größe $\sigma$. Um einen kleinen Winkel zwischen Laserstrahl 15 und Beobachtungsrichtung 14 zu erreichen und außerdem eine flexible Zugänglichkeit zu den gewünschten Meßpunkten zu gewährleisten, werden Lichtleiter 7, 8, 10 eingesetzt. Der Strahl aus dem Laser 1 wird durch die Linse 2 in den Lichtleiter 3 fokussiert. Der austretende Strahl divergiert stark, so daß er mit der Linse 4 zum Meßpunkt 5 auf dem Objekt 6 gebündelt wird. Der Durchmesser D des Strahlflecks im Meßpunkt 5 muß möglichst klein sein, damit die Speckle-Größe $\sigma$ eine zur Detektion erforderliche Mindestgröße erreicht. Der Abstand zwischen Meßpunkt 5 und dem Beobachtungsort 16 darf andererseits nicht zu groß sein, weil sonst die zur Verfügung stehende Lichtintensität für eine Detektion nicht ausreicht. Andererseits darf der Durchmesser D nicht stark vom Abstand Linse 4 - Objekt 6 abhängen, weil sonst schon eine geringe Variation des Abstandes zu einer großen Änderung der Speckle-Größe $\sigma$ führt, die wiederum eine fehlerhafte Messung der Geschwindigkeit zur Folge hat. Das Strahlprofil soll daher eine lange schmale Taille 17 haben.

Zur Detektion dient eine Einzelfaser 10 des Faser-Bündels 7 am Beobachtungsort 16, das möglichst nahe neben dem Ende des Lichtleiters 3 und der Linse 4 montiert ist, um den Winkel zwischen auftreffendem Laser 15 und Beobachtungsrichtung 14 gering zu halten. Da der wirksame Durchmesser der Einzelfaser 10 die effektive Blende 30 für die Speckle-Detektion ist, muß der Abstand zwischen Meßpunkt 5 und Beobachtungsort 16 so gewählt sein, daß die Speckle-Größe $\sigma$ etwas größer ist als der Faser-Durchmesser.

Bei einer Objektbewegung ziehen die Speckles an dem Ende der Einzelfaser (10) vorbei, so daß der Lichtdetektor (11, z.B. ein Fotomultiplier), zu dem die Einzelfaser (10) führt, die zeitabhängige Speckle-Intensität I registriert. Die Einzelfaser (10) liegt in der Mitte des Faserbündels (8), das zu einem weiteren Detektor 9 führt. Da das Faserbündel 8 aus etwa 400 Einzelfasern mit dem Durchmesser der Speckle-Größe $\sigma$ besteht, und da das eintreffende Licht in einem gemeinsamen Detektor 9 gemessen wird, ist der Meßwert die mittlere Speckle-Intensität $\bar{I}$ an demselben Ort, an dem auch die Speckle-Intensität I gemessen wird. Die Güte der Mittelwertbildung G ist proportional zu $\sqrt{N}$ .

Der Tubus 18 dient zur Einhaltung des optimalen Abstandes zwischen Meßkopf 19 und Objekt 6. Der Filter 12 läßt nur Licht der Laser-Wellenlänge passieren und vermindert so das Rauschen der Detektoren 9 und 11, das durch Streulicht anderer Wellenlängen verursacht wird.

I ist damit das zeitabhängige Signal der Speckle-Intensität, dessen Spektrum durch das räumliche Spektrum des Speckle-Musters und die Objektgeschwindigkeit bestimmt ist.

Voraussetzung für die Anwendung des erfindungsgemäßen Verfahrens ist, daß die Intensität I(t) mit einem Detektor 11 ermittelt wird, dessen effektive Fläche kleiner ist als die mittlere Speckle-Größe. Die effektive Fläche kann durch den Detektor selbst, eine kleine vorgesetzte Blende oder den Querschnitt des vorgesetzten Lichtleiters definiert werden.

Die Bestimmung der Geschwindigkeit hängt im Gegensatz zum Verfahren des Speckle-Counting nicht empfindlich ab von der Gesamtintensität des Streulichts. Es ist deshalb im Prinzip nicht notwendig, die Gesamtintensität zu bestimmen. Wenn die Gesamtlichtintensität nur um 5 % schwankt, hat auch die ermittelte Geschwindigkeit einen Fehler von 5 %. Aber auch diese Fehlermöglichkeit kann kompensiert werden.

Ein gleichzeitiges Messen der Laserlichtintensität und Division eliminiert Einflüsse der Schwankungen der Laserlichtintensität. Ein gleichzeitiges Messen der Gesamtintensität des Streulichts und Division eliminiert auch Einflüsse, die durch Reflexionsänderungen des Objekts 6 hervorgerufen werden.

Eine gleichzeitige Messung der Gesamtintensität des Streulichts $\bar{I}$ am selben Ort wie die Bestimmung

4

der Speckle-Intensität I eliminiert auch Einflüsse der richtungsabhängigen Streuung.

I und Ī können auf verschiedene Weise bestimmt werden, z.B. durch einen Beam-Splitter oder durch die Glasfaser-Anordnung. Der Nutzen der Glasfaser-Anordnung liegt in ihrer guten Handhabbarkeit, was insbesondere bei Messungen an der Haut zum Tragen kommt.

Wenn bei einem stehenden Speckle-Muster mit einer kleinen Blende 30 das Speckle-Muster abgetastet wird, entsteht eine Ortsabhängigkeit $I_r(Y)$ mit Ortsspektrum. Das Ortsspektrum des Speckles ist aus der Literatur allgemein bekannt. Bewegt sich nun das Speckle-Muster (Sinus-Gitter) mit der Geschwindigkeit V und wird an einer Stelle beobachtet, so entsteht eine zeitabhängige Lichtintensität I(t) hinter der Blende mit $I(t) = I_r(vt)$. Zur Illustration der Vorgänge beim Speckle-Muster wird eine sinus-förmige Lichtintensitätsverteilung betrachtet, die mit der Geschwindigkeit $V_S$ an der Blende vorbeigeführt wird. Die Sinus-Intensität hat die Ortsabhängigkeit

$$I_{i,t}(Y) = I_o \sin \left(2\pi \frac{Y}{Y_o}\right).$$

Die entsprechende Zeitabhängigkeit ist

$$I_i(t) = I_o \sin \left(2\pi \frac{vt}{Y_o}\right).$$

Offensichtlich führt eine hohe Geschwindigkeit in beiden zeitabhängigen Funktionen zu einem Auftreten höherer Frequenzen.

Damit die Geschwindigkeit in die Amplitude eingeht, wird einmal zeitlich differenziert (siehe Differenzierglied in der elektrischen Schaltung).

$$\dot{I}(t) = d\,dt\,I_r(vt)$$

$$\text{z.B. } \dot{I}_i(t) = 2\pi \frac{V}{Y_o} I_o \cos \left(2\pi \frac{vt}{Y_o}\right)$$

Nun wird die Intensität des Signals $\dot{I}(t)$ bestimmt, um die Geschwindigkeit zu erhalten.

$$P(v) = \sqrt{\int / I(t)/^2 \, dt}$$

Für das Ausführungsbeispiel kann ausgerechnet werden:

$$P_i(v) = \sqrt{\int_o^\infty / 2\pi \frac{V}{Y_o} I_o \cos(2\pi \frac{vt}{Y_o})/^2 \, dt}$$

$$= \sqrt{\frac{1}{2} \left(2\pi \frac{V}{Y_o} I_o\right)^2}$$

$$= \sqrt{2}\, \pi \frac{V}{Y_o} I_o$$

$$M(v) = \frac{P(v)}{I_o} = \sqrt{2}\, \pi \frac{V}{Y_o} \qquad (\text{der Meßwert})$$

Für das Speckle-Muster läßt sich der Wert P(v) nur im statistischem Mittel ausrechnen, weil das Ortsspektrum der Speckles (im statistischem Mittel) bekannt ist.

Nach einem Theorem gilt (Power-Theorem)

$$\int /\dot{I}(t)/^2 dt = \int / \mathcal{T}(\dot{I}(t))/^2 d\nu$$

Das ist das Quadrat der Fourier - Transformation von I(t), das "Power-Spektrum".

$$\int /I(t)/^2 dt$$

$$= \int / \tau\ (\dot{I}(t))/^2 d\nu$$

$$= \int / \tau\ (\tfrac{d}{dt}\ I_r(vt))/^2 d\nu$$

$$= \int / 2\pi i\ \tfrac{\nu}{v}\ \tau(I_r(vt))/^2 d\nu$$

denn: zeitliche Differentation entspricht im Spektrum einer Multiplikation mit der Frequenz. $\tfrac{1}{v}$ entsteht durch eine Art Substituionsregel.

$$= 4\pi^2\ \int \nu^2\ /\tfrac{1}{v}\ \mathcal{T}(I_r(vt))/^2 d\nu$$

Das ist das aus der Literatur bekannte Spektrum des räumlichen Speckle-Musters. Das wird eingesetzt.

$$= 4\pi^2\ \int \nu^2\ (\ \bar{I}^2(-\frac{\sigma^2}{v^2}\ \nu + \frac{\sigma}{v})\ d\nu$$

wobei $\bar{I}$ die mittlere Intensität und $\sigma$ die mittlere Speckle-Größe ist.

$$= \tfrac{2}{3}\ \pi^2\ I_o^2\ \frac{v^2}{\sigma^2}$$

Das ergibt:

$$P(v) = \sqrt{\tfrac{2}{3}}\ \pi\ I_o\ \frac{v}{\sigma}$$

$$\boxed{M(v)\ =\ \sqrt{\tfrac{2}{3}}\ \pi\ \frac{v}{\sigma}}$$

Ein Vergleich mit dem Ergebnis des Sinus-Gitters ergibt

$$Y_o \cong \sqrt{3}\ \sigma$$

Die zeitliche Mittelung, d.h. die Intensitätsbestimmung entspricht einer Integration des Spektrums. Sind zwei Geschwindigkeiten überlagert , kann durch eine geschickte Auswahl der Frequenzintervalle eine

6

Trennung erfolgen. Wenn nun für die Intensitätsbildung nur angegebene Frequenzintervalle ausgenutzt werden, erfolgt eine Trennung der Geschwindigkeitsmessungen. Die Realisierung besteht darin, daß das zeitabhängige Signal durch aktive Filter verändert wird. So ist es bekannt, daß ein Hochpaß die niedrigen Frequenzen unterdrückt und damit zur Detektion hoher Geschwindigkeiten dient (entsprechendes gilt für den Tiefpaß bzw. niedrige Geschwindigkeiten).

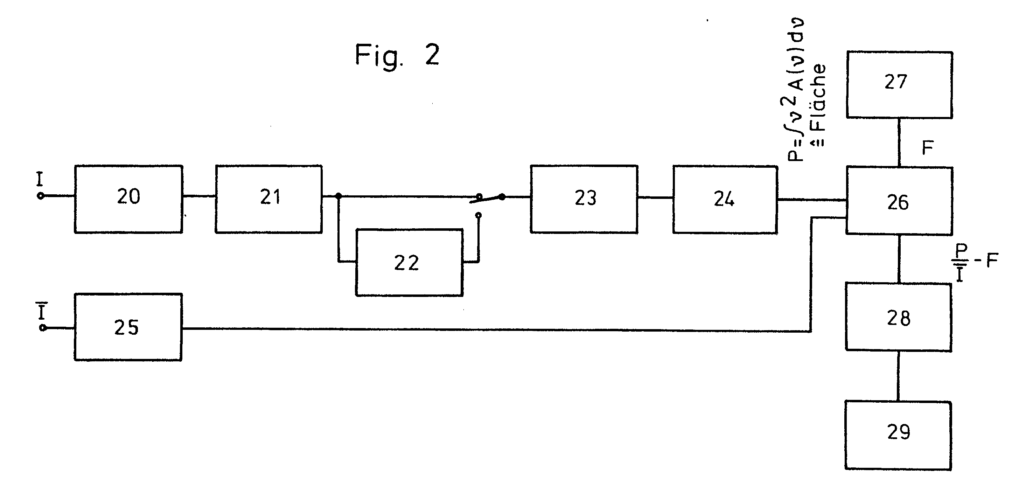

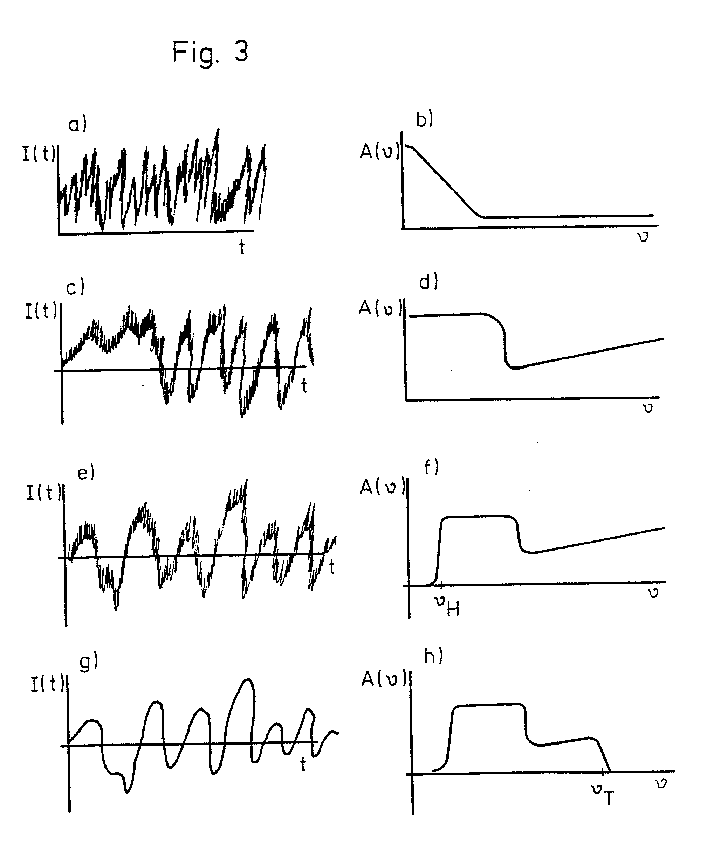

Die Realisierung des Meßwertes $M(V_s)$ kann durch die in Figur 2 dargestellte elektronische Schaltung erfüllt werden, wobei in Figur 3 a bis h die Wirkungsweise der einzelnen Komponenten der Schaltungsanordnung dargestellt ist.

Das Signal I wird durch den Verstärker 20 verstärkt (siehe Zeitabhängigkeit des verstärken Eingangssignals sowie das Spektrum in Figur 3 a und b) und passiert den Linearpaß 21 bzw. ein Differenzierglied, in dem eine Verstärkung proportional zur Frequenz $v$ mit einer einstellbaren Proportionalitätskonstanten c erfolgt (Ausgangssignal und Spektrum in Figur 3c und d). Die einfachste Realisierungsmöglichkeit besteht in einem RC-Glied als passivem Bauelement. Höhere Ansprüche an die Linearität und Dynamik kann eine aktive Schaltung erfüllen.

Dem Speckle-Signal ist das Rauschen von Fotomultiplier und Laser überlagert. Der aktive Tiefpaß-Filter 23 dient zum Einengen des betrachteten Frequenzbereichs z.B. bis zur Grenzfrequenz $v_T$ ohne die Linearität zu beanträchtigen, falls die Grenzfrequenz $v_T$ größer ist als die Maximalfrequenz in I, die durch die Speckle-Größe $\sigma$ und die Maximalgeschwindigkeit festgelegt ist (Ausgangsignal des Tiefpasses 23 sowie Spektrum siehe Figur 3 g und h).

Der RMS-DC-Converter 24 führt praktisch eine Integration durch und ermittelt die Gesamtintensität des zeitabhängigen Signals. Die Integration ist im Prinzip eine "spezielle" zeitliche Integration des zeitabhängigen Signals. Deutlicher ist es aber im Spektrum zu sehen, in dem eine Frequenz-Integration über ein bestimmtes Frequenzintervall erfolgt. Dieser Wert wird im Dividierer 26 durch das im parallel liegenden Verstärker 25 verstärkte Signal der gleichzeitig gemessenen mittleren Intensität $\bar{I}$ dividiert und stellt dann den Meßwert $M(V_S)$ dar. $M(V_S)$ wird nach einer zeitlichen Integration im Integrator 28, welche die Genauigkeit der Messung bestimmt, im Anzeigegerät 29 angezeigt.

Das Rauschen von Fotomultiplier und Laser tritt natürlich auch in dem betrachteten Frequenzbereich auf. Dieser Anteil am Meßsignal $M(V_S)$ kann durch einen Offset 27 eliminiert werden, indem vom Quotienten ein fester einstellbarer Betrag subtrahiert wird.

Da im höherfrequenten Anteil ein Einfluß der Blut-Flußbewegung am deutlichsten zu erkennen ist, ist die mittlere Geschwindigkeit der unwillkürlichen Körperbewegung geringer als die der Blutbewegung. Dieser Unterschied wird zur Separation beider Geschwindigkeiten ausgenutzt. Dazu dient der einschaltbare aktive Hochpaß-Filter 22, dessen Ausgangssignal bzw. Spektrum für die Grenzfrequenz $\gamma_H$ in Figur 3 e und f dargestellt ist. Dieser Filter 22 muß einerseits die beiden Geschwindigkeitsbereiche scharf trennen, d.h. er muß eine scharfe Kante haben, andererseits muß er zur Erhaltung der Linearität im Durchlaßbereich frequenzunabhängig sein.

Mit der Schaltungsanordnung bzw. der Einrichtung nach Figur 1 erfolgt bei Variation der Beobachtungsebene um ± 5 mm keine Änderung des Geschwindigkeitssignals. Die Linearität des Verfahrens ist in Figur 4 dargestellt. Die Abweichungen bei kleinen Geschwindigkeiten sind darauf zurückzuführen, daß der Strahlfleck nicht wie in der theoretischen Ableitung quadratisch ist, sondern ein $e^{-x^2}$ - Profil hat, und daß die Speckle-Bewegung mit einer Blende gemessen wird, die nicht vernachlässigbar klein ist im Vergleich zur Speckle-Größe. Die Zeitabhängigkeit der Durchblutung bei unterdrückter Blutzufuhr während des Zeitintervalls$\Delta$ T wird in Figur 5 dargestellt und die Änderung der Durchblutung bei Anwendung einer durchblutungsfördernden Salbe ist in Figur 6 aufgezeigt.

Es kann sich in der Praxis als sinnvoll erweisen, daß bei der Bestimmung einer Größe B die den Blutfluß angibt, andere Wichtungen zu besseren (besser bedeutet unterscheidbaren bzw. stabileren) Meßwerte führen als die jetzt angegebenen. Ein Wichtung proportional zu $v^2$ oder $v^3$ würde besonders den Anteil hoher Geschwindigkeiten hervorheben. Außerdem kann durch einen stufenförmigen Filter, einen geeigneten Hochpaß-Filter, der einerseits im Spektrum die Frequenzen nahe O unterdrückt (die durch den Gleichlicht-Anteil in der Speckle-Intensität *) andererseits den Rest frequenzunabhängig verstärkt, ein Meßsignal erzeugt werden, das z.B. den Anteil der Blutbewegung unabhängig von der Geschwindigkeit anzeigt.

Diese Wichtungen lassen sich mit einer modifizierten Schaltungsanordnung realisieren. Die Verstärkung proportional zu $v^2$ entspräche dabei einer 2-maligen Differentiation und eine Verstärkung proportional zu $v^3$ bedürfte eines speziellen Verstärkers mit dieser Kennlinie. Vor und nach der Power-Bildung (Power-Meter 24 nach Figur 2) müßte dann quadriert werden. Auch das Elektronikelement 21 müßte dann verallgemeinert

*)hervorgerufen     werden),

7

werden und zwar zu einem Element, das das Signal frequenzgewichtet verstärkt.

Messungen mit dem erfindungsgemäßen Verfahren zeigten, daß Geschwindigkeiten bis zu einigen 100 µm/s ermittelt werden können.Diese untere Grenze ist durch die zur Verfügung stehende Meßblende 30 von einigen µm Durchmesser D gegeben, da auf diese Weise eine Mindestgröße der Speckles festgelegt ist. Zur Messung von höheren Geschwindigkeiten ist ein Vergrößern des erfaßten Frequenzbereichs notwendig, so daß das Rauschen ansteigt. Das wiederum läßt sich durch ein Erhöhen der Laserintensität ausgleichen.

**Ansprüche**

1. Verfahren zur berührungslosen real-time-Bestimmung von Geschwindigkeiten einer oder mehrerer Bewegungen an einem Objekt in senkrechter Richtung zu einer auf das Objekt auftreffenden kohärenten Strahlung, deren Streulicht ein räumliches Speckle-Muster mit einem Speckle-Spektrum erzeugt, wobei das Speckle-Muster durch die Bewegungen am Objekt zeitabhängig wird, dadurch gekennzeichnet, daß ein geschwindigkeits-proportionaler Meßwert M ($V_s$) durch die Bestimmung einer Intensität des frequenzabhängig gewichteten zeitabhängigen Speckle-Intensitätssignals I ermittelt wird.

2. Verfahren nach Anspruch 1, dadurch gekennzeichnet, daß durch die Verwendung verschiedener Frequenzintervalle bei der Wichtung unterschiedliche Objektgeschwindigkeiten voneinander getrennt werden und bei gleichzeitiger Unterdrückung der Frequenzen bei O der Anteil einer einzelnen Bewegung ermittelt wird.

3. Verfahren nach Anspruch 1 und 2, dadurch gekennzeichnet, daß die Wichtung proportional zur Frequenz γ oder zur potenzierten Frequenz erfolgt.

4. Schaltungsanordnung zur Ermittlung eines geschwindigkeitsproportionalen Meßwertes M($V_s$) unter Verwendung eines Verfahrens gemäß einem der Ansprüche 1-3, gekennzeichnet durch
   a) eine Parallelschaltung eines Verstärkers (20) für das Speckle-Intensitätssignal I, der in Reihe mit einem Linearpaß (21), einem Tiefpaß-Filter (23) und einem Integrator (24) liegt, mit einem weiteren Verstärker (25) für eine mittlere Intensität (Ī ),die der Gesamtintensität des Streulichts am Ort der Messung der Speckle-Intensität, entspricht,
   b) eine Verknüpfung beider verarbeiteten Intensitätswerte (I und Ī ) in einem Dividierer (26) und
   c) eine zeitliche Integration des Wertes aus dem Dividierer (26) in einem Integrator (28).

5. Schaltungsanordnung nach Anspruch 4, gekennzeichnet durch einen Off-set (27) am Dividierer (26).

6. Schaltungsanordnung nach Anspruch 4 und/oder 5, gekennzeichnet durch einen zwischen Linearpaß (21) und Tiefpaß (23) einschaltbaren Hochpaßfilter (22).

7. Einrichtung zur Durchführung der Messungen der Speckle-Intensität und der mittleren Intensität für die Ermittlung eines geschwindigkeitsproportionalen Meßwertes M($V_s$) in einer Schaltungsanordnung gemäß einem der Ansprüche 4-6, dadurch gekennzeichnet, daß die Aufnahme des räumlichen Speckle-Spektrums mittels eines Faserbündels (7) erfolgt, wobei die Erfassung der Speckle-Intensität (I) mit einer der Fasern (10) und die Erfassung der mittleren Intensität (Ī ) mittels der restlichen Fasern (8) durchführbar ist.

8. Verwendung des Verfahrens nach einem der Ansprüche 1-3, der Schaltungsanordnung nach einem der Ansprüche 4-6 sowie der Einrichtung nach Anspruch 7 zur Ermittlung der Blutbewegung an oder in Gewebe, beispielsweise an Haut, in Wunden oder anderen von außen zugänglichen Körperstellen unabhängig von Körperbewegungen.

**Claims**

1. Method for the contactless and real-time determination of speeds of one or more movements at an

8

EP 0 150 035 B1

object in a vertical direction relative to a coherent radiation, which impinges upon the object and the stray light of which produces a three-dimensional speckle pattern utilising a speckle spectrum, the speckle pattern becoming time-dependent as a result of the movements on the object, characterised in that a speed-proportional measurement value $M(V_s)$ is ascertained by the determination of an intensity of the time-dependent speckle intensity signal I, which is loaded in dependence on the frequency.

2. Method according to claim 1, characterised in that, by using various frequency intervals during the loading, different object speeds are separated from one another and, with the simultaneous suppression of the frequencies at 0, the proportion of an individual movement is ascertained.

3. Method according to claims 1 and 2, characterised in that the loading is effected in proportion to the frequency $\gamma$ or the frequency which has been raised to a higher power.

4. Circuit arrangement for ascertaining a speed-proportional measurement value $M(V_s)$, using a method in accordance with one of claims 1 - 3, characterised by
   a) a parallel circuit of an amplifier (20) for the speckle intensity signal I, which amplifier lies in series with a linear-pass filter (21), a low-pass filter (23) and an integrator (24), with an additional amplifier (25) for an average intensity ($\bar{I}$), which corresponds to the total intensity of the stray light at the location where the speckle intensity is measured;
   b) a combination of both processed intensity values (I and $\bar{I}$) in a divider (26); and
   c) an integration, per unit time, of the value from the divider (26) in an integrator (28).

5. Circuit arrangement according to claim 4, characterised by an off-set (27) at the divider (26).

6. Circuit arrangement according to claim 4 and/or 5, characterised by a high-pass filter (22), which is insertable between linear-pass filter (21) and low-pass filter (23).

7. Apparatus for carrying out the measurements of the speckle intensity and the average intensity for ascertaining a speed-proportional measurement value $M(V_s)$ in a circuit arrangement in accordance with one of claims 4 - 6, characterised in that the three-dimensional speckle spectrum is picked-up by means of a fibre bundle (7), the speckle intensity (I) being detectable using one of the fibres (10), and the average intensity ($\bar{I}$) being detectable by means of the remaining fibres (8).

8. Use of the method according to one of claims 1 - 3, the circuit arrangement according to one of claims 4 - 6 and the apparatus according to claim 7 for determining the movement of blood on or in tissue, for example on skin, in wounds or at other locations of the body, which are accessible from externally, independently of body movements.

**Revendications**

1. Procédé pour la détermination sans contact, en temps réel, des vitesses d'un ou plusieurs déplacements sur un objet, perpendiculairement à un rayonnement cohérent incident sur cet objet, et dont la lumière diffusée engendre un motif spatial de minuscules spots (speckles) avec un spectre de sports, le motif de spots variant en fonction du temps du fait des déplacements sur l'objet, procédé caractérisé en ce qu'une valeur de mesure M ($V_s$) proportionnelle à la vitesse est obtenue en déterminant une intensité du signal d'intensité des spots, pondéré en fréquence et variant en fonction du temps.

2. Procédé selon la revendication 1, caractérisé en ce qu'en utilisant différents intervalles de fréquence lors de la pondération, différentes vitesses de l'objet sont séparées les unes des autres, et en réduisant simultanément les fréquences à 0, la part afférente à un déplacement particulier est déterminée.

3. Procédé selon la revendication 1 et la revendication 2, caractérisé en ce que la pondération s'effectue proportionnellement à la fréquence $\gamma$ ou à la fréquence élevée à une certaine puissance.

4. Agencement de circuit pour déterminer une valeur de mesure M($V_s$) proportionnelle à la vitesse, en utilisant un procédé selon une des revendications 1 à 3, agencement de circuit caractérisé en ce qu'il comporte :

9

a) un branchement en parallèle d'un amplificateur (20) pour le signal d'intensité (I) des spots, qui est placé en série avec un filtre linéaire (21), un filtre passe-bas (23) et un intégrateur (24), avec un autre amplificateur (25) pour une intensité moyenne (I) qui correspond à l'intensité totale de la lumière diffusée à l'endroit de la mesure de l'intensité des spots,

b) une combinaison de deux valeurs d'intensité traitées (I et I) dans un diviseur (26),

c) une intégration en fonction du temps de la valeur en provenance du diviseur (26) dans un intégrateur (28).

5. Agencement de circuit selon la revendication 4, caractérisé en ce qu'il comporte un décaleur de fréquence (27) sur le diviseur (26).

6. Agencement de circuit selon la revendication 4 et/ou la revendication 5, caractérisé en ce qu'il comporte un filtre passe-haut (22) susceptible d'être branché entre le filtre linéaire (21) et le filtre passe-bas (23).

7. Dispositif pour effectuer les mesures de l'intensité des spots et de l'intensité moyenne, pour la détermination d'une valeur de mesure $M(V_s)$ proportionnelle à la vitesse, dans un agencement de circuit selon une des revendications 4 à 6, dispositif caractérisé en ce que le spectre spatial des spots est capté au moyen d'un faisceau de fibres (7), la détection de l'intensité (I) des spots étant susceptible d'être réalisée avec l'une (10) des fibres, tandis que la détection de l'intensité moyenne (I) est susceptible d'être réalisée avec les fibres restantes (8).

8. Utilisation du procédé selon une des revendications 1 à 3, de l'agencement de circuit selon une des revendications 4 à 6, ainsi que du dispositif selon la revendication 7 pour déterminer le déplacement du sang sur ou dans un tissu, par exemple sur la peau, dans des blessures ou d'autres emplacements du corps, de façon indépendante des mouvements du corps.

Fig. 1

Fig. 2

20 21 22 23 24 25 26 27 28 29

$$P = \int v^2 A(v)\, dv \; \hat{=} \; \text{Fläche}$$

F

$$\frac{P}{I} - F$$

EP 0 150 035 B1

# Fig. 3

Fig. 4

Fig. 5

Fig. 6